Europäisches Patentamt

European Patent Office

Office européen des brevets

(11) Veröffentlichungsnummer: **0 283 539 B1**

# EUROPÄISCHE PATENTSCHRIFT

(45) Veröffentlichungstag der Patentschrift: **29.05.91**

(51) Int. Cl.⁵: **A61M 25/00**

(21) Anmeldenummer: **87104488.9**

(22) Anmeldetag: **26.03.87**

(54) Inplantierbarer, arterieller Katheter insbesondere Zuspritzkatheter.

(43) Veröffentlichungstag der Anmeldung:
28.09.88 Patentblatt 88/39

(45) Bekanntmachung des Hinweises auf die
Patenterteilung:
29.05.91 Patentblatt 91/22

(84) Benannte Vertragsstaaten:
AT BE CH DE ES FR GB GR IT LI LU NL SE

(56) Entgegenhaltungen:
EP-A- 0 183 351       WO-A-83/01387
DE-A- 2 337 717       DE-A- 3 539 022
DE-A- 3 605 460       US-A- 3 109 429
US-A- 4 475 898

(73) Patentinhaber: **Clinical Plastic Products SA
5, rue L. J. Chevrolet
2300 La Chaux-de-Fonds(CH)**

(72) Erfinder: **Jeanneret, Hedwig
Helvétie 20
CH-2300 La Chaux-de-Fonds(CH)**

(74) Vertreter: **Hanewinkel, Lorenz, Dipl.-Phys.
Patentanwalt Ferrariweg 17a
W-4790 Paderborn(DE)**

## Beschreibung

Die Erfindung bezieht sich auf einen implantierbaren, arteriellen Katheter, insbesondere Zuspritzkatheter, mit einem aus einer Schale mit deren offenen Schalenseite dicht verschließenden, durchstechbaren Membrane und einem Fixierrand bestehenden Zuspritzteil (Port) und einem am Zuspritzteil mit einem Ende befestigten Katheterschlauch, der am anderen Ende ein Rückschlagventil aufweist. Ein derartiger Katheter ist aus der EP-A-0183351 bekannt.

Bei derartigen bekannten Kathetern ist das Rückschlagventil vom geschlossenen Katheterschlauchende mit seitlicher Lochung und einer darüber geschobenen Schutzkappe gebildet, so daß durch den Flüssigkeitsdruck die Schutzkappe im Bereich der seitlichen Lochung aufgeweitet wird und die Flüssigkeit ausströmen kann.

Aufgabe der Erfindung ist es, einen nach dem Oberbegriff des Patentanspruches 1 aufgebauten Katheter mit einem in der Funktion und Sicherheit verbesserten Rückschlagventil auszustatten, das neben einer einfachen Ausführung einen guten Flüssigkeitsaustritt ermöglicht und eine sehr hohe Rückschlagwirkung hat.

Diese Aufgabe wird erfindungsgemäß dadurch gelöst, daß das Rückschlagventil von einem am Stirnende des Katheterschlauches angeordneten Spitzkegel mit in der Kegelspitze vorgesehenen Durchflußöffnungen und einer den Spitzkegel und das Katheterschlauchende übergreifenden, auf den Katheterschlauch festgelegten und im Spitzkegelbereich stirnseitig eine Durchflußöffnung aufweisenden Schutzkappe gebildet ist.

In bevorzugter Weise sind die beiden in Katheterschlauchlängsrichtung mit Abstand koaxial hintereinanderliegenden Durchflußöffnungen in dem Spitzkegel und der Schutzkappe von Schlitzen gebildet. Der aus einem weicheren Kunststoffmaterial als die Schutzkappe bestehende Spitzkegel läßt sich durch Kleben, Anvulkanisieren od. dgl. am Stirnende des Katherschlauches befestigen oder aber einstückig an dem Katheterschlauch anformen.

Weitere Merkmale der Erfindung sind aus den übrigen Unteransprüchen zu entnehmen.

Der Gegenstand der Erfindung erstreckt sich nicht nur auf die Merkmale der einzelnen Ansprüche, sondern auch auf deren Kombination.

Der implantierbare Katheter ist erfindungsgemäß mit einem Rückschlagventil ausgestattet, das einerseits eine gute Ausflußmöglichkeit für die aus dem Katheterschlauch austretende Flüssigkeit hat und andererseits eine sehr hohe Rückschlagwirkung gegen Eintritt von Blut od. dgl. zeigt, so daß ein möglicher thrombotischer Verschluß ausgeschlossen wird.

Dieses Rückschlagventil setzt sich aus einem am Stirnende des Katheterschlauches angeordneten Spitzkegel mit in der Kegelspitz vorgesehener Durchflußöffnung und einer den Spitzkegel und das Katheterschlauchende übergreifenden Schutzkappe mit stirnseitiger Durchflußöffnung zusammen, so daß die beiden Durchflußöffnungen in Schlauchöffnungen koaxial hintereinander liegen. Die beiden Durchflußöffnungen werden in Abhängigkeit von dem Druck der aus dem Katheterschlauch austretenden Flüssigkeit geöffnet, und zwar nacheinander mit Verzögerung, wobei zuerst die Flüssigkeit aus dem Spitzkegel austritt und dann durch ihren Druck die Durchflußöffnung in der Kappe zum Flüssigkeitsaustritt öffnet, wodurch die Flüssigkeit praktisch in zwei nacheinander folgenden Stufen aus dem Rückschlagventil austritt.

Bei nachlassendem Flüssigkeitsdruck wird sofort die Öffnung im Spitzkegel geschlossen und danach die Öffnung in der Schutzkappe, was eine Doppelwirkung inder Rückschlagsicherung ergibt und damit einen Rückstrom von Blut od. dgl. ausschließt.

Dieses Rückschlagventil ist einfach aufgebaut und zeigt eine lange Lebensdauer bei sehr sicherer Wirkungsweise.

Anhand der Zeichnung wird nachfolgend ein Ausführungsbeispiel der Erfindung näher erläutert. Es zeigt:

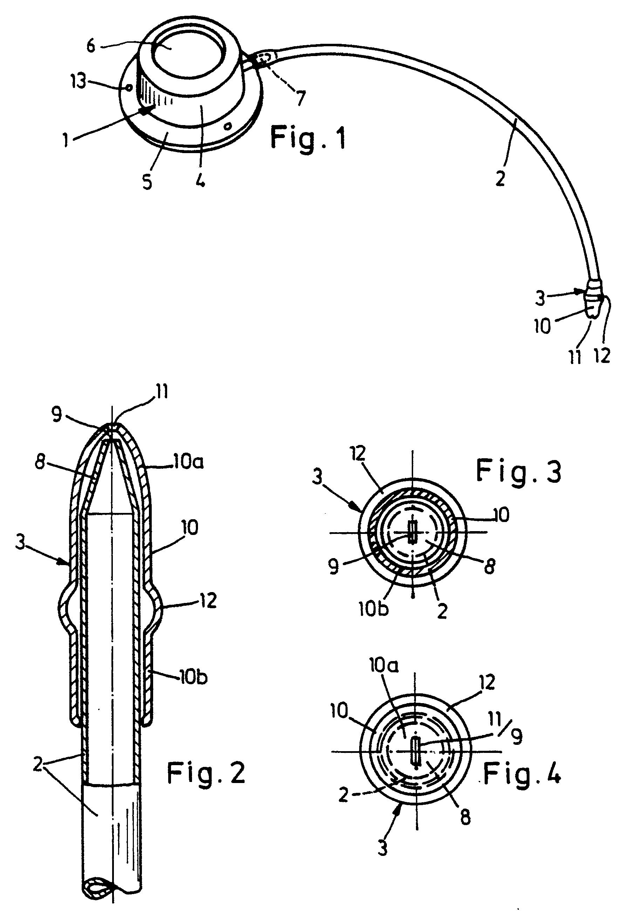

Fig. 1    eine perspektivische Ansicht eines implantierbaren, arteriellen Katheters mit Zuspritzteil und daran festgelegtem Katheterschlauch mit endseitigem Rückschlagventil

Fig. 2    einen Längsschnitt durch das am Katheterschlauch angeordnete Rückschlagventil mit am Schlauch vorgesehenem Spitzkegel und diesen übergreifender Schutzkappe;

Fig. 3    einen Querschnitt durch das Sicherheitsventil mit geschnittener Schutzkappe und nicht geschnittenem Spitzkegel;

Fig. 4    eine Draufsicht auf das gesamte Sicherheitsventil.

Ein implantierbarer, arterieller Katheter, insbesondere Zuspritzkatheter, besitzt ein Zuspritzteil 1, an dem ein Katheterschlauch 2 mit endseitigem Rückschlagventil 3 angeschlossen ist. Das Zuspritzteil 1 setzt sich aus einer Schale 4 mit einem am Schalenboden nach außen vorstehenden Fixierrand 5 und einer die offene Schalenseite verschließenden durchstechbaren Membrane 6 zusammen. Dieses Zuspritzteil 1 (Port) wird mittels seines Fixierrandes 5 und darin in bekannter Weise ausgesparter Löcher (13) durch eine chirurgische Naht an der Faszie befestigt, so daß die implantierte Lage im Körper fixiert ist. Der Katheterschlauch 2 ist mit einem Längenende fest auf einem seitlichen Stutzen 7 des Zuspritzteiles 1 befestigt und weist an seinem anderen Längenende das Rückschlagventil 3 auf.

Das Rückschlagventil 3 besteht aus einem am Stirnende des Katheterschlauches 2 angeordneten Spitzkegel 8 mit einer in der Kegelspitze vorgesehenen Durchflußöffnung 9 und einer den Spitzkegel 8 und das Katheterschlauchende übergreifenden, auf dem Katheterschlauch festgelegten Schutzkappe 10, die stirnseitig ebenfalls eine Durchflußöffnung 11 aufweist.

Die beiden in Katheterschlauchlängsrichtung im Abstand koaxial hintereinander liegenden Durchflußöffnungen 9, 11 sind von in Abhängigkeit von der durch das Rückschlagventil 3 austretenden Flüssigkeit selbsttätig auf- und zugehenden Schlitzen gebildet.

Die Schutzkappe 10 ist vom spitzkegelseitigen Ende des Katheterschlauches 2 auf denselben aufgeschoben und auf den Katheterschlauch 2 festgelegt und an ihrem freien Stirnende bis auf die Durchflußöffnung 11 geschlossen. Dabei zeigt die Schutzkappe 10 im Bereich des Spitzkegels 8 eine parabelförmige Körperform, die etwa der gerundeten Kopfform eines Geschosses entspricht, und an diese parabelförmige Körperform (dem Kappenkopf 10a) schließt sich ein zylindrischer, über den Katheterschlauch 2 auf einer gewissen Länge greifender Kappenmantel 10b an, der eine nach außen gewölbte Fixierwulst 12 zur Fixierung des Katheterschlauches 2 mit seinem Rückschlagventil 3 im Gefäß (Arterie/Vene) hat.

Der parabelförmige Kappenkopf 10a übergreift den Spitzkegel 8 mit geringem Abstand und die beiden schlitzförmigen Durchflußöffnungen 9, 11 liegen in Katheterschlauchlängsrichtung mit geringem Abstand koaxial hintereinander.

Der Spitzkegel 8 läßt sich als gesondertes Teil an der Stirnseite des Katheterschlauches 2 befestigen und zwar durch Kleben oder Anvulkanisieren; weiterhin besteht die Möglichkeit, den Spitzkegel 8 an den Katheterschlauch 2 einstückig anzuformen. Der Spitzkegel 8 besteht aus einem weicheren Material als die Schutzkappe 10.

Das Zuspritzteil 1 und der Katheterschlauch 2 mit Rückschlagventil 3 bestehen aus einem geeigneten Kunststoff.

Die durch eine Injektion in das Zuspritzteil 1 eingebrachte und durch den Katheterschlauch 2 fließende Flüssigkeit tritt durch das Rückschlagventil 3 in das Gefäß aus. Dabei wird die geschlossene Durchflußöffnung 9 im Spitzkegel 8 durch den Druck der Flüssigkeit geöffnet und die Flüssigkeit kann in die Schutzkappe 10 im Spitzkegelbereich eintreten und dann wird mit Verzögerung durch den Druck der Flüssigkeit auch die Durchflußöffnung 11 der Schutzkappe 10 geöffnet und die Flüssigkeit tritt aus. Bei nachlassendem Flüssigkeitsdruck schließen sich die Durchflußöffnungen 9, 11 automatisch und dadurch wird ein Rückstrom von Blut od. dgl. sicher verhindert.

## Ansprüche

1. Implantierbarer, arterieller Katheter, insbesondere Zuspritzkatheter, mit einem aus einer Schale mit deren offenen Schalenseite dicht verschließenden, durchstechbaren Membrane und einem Fixierrand bestehenden Zuspritzteil (Port) und einem am Zuspritzteil mit einem Ende befestigten Katheterschlauch, der am anderen Ende ein Rückschlagventil aufweist, dadurch gekennzeichnet, daß das Rückschlagventil (3) von einem am Stirnende des Katheterschlauches (2) angeordneten Spitzkegel (8) mit in der Kegelspitze vorgesehener Durchflußöffnung (9) und einer den Spitzkegel (8) und das Katheterschlauchende übergreifenden, auf den Katheterschlauch (2) festgelegten und im Spitzkegelbereich stirnseitig eine Durchflußöffnung (11) aufweisenden Schutzkappe (10) gebildet ist.

2. Katheter nach Anspruch 1, dadurch gekennzeichnet, daß die Durchflußöffnung (9) im Spitzkegel (8) und die Durchflußöffnung (11) in der Schutzkappe (10) von in Abhängigkeit von der über das Zuspritzteil (1) in den Katherschlauch (2) eingebrachten Flüssigkeit durch den Flüssigkeitsdruck auf-und zugehenden Schlitzen gebildet sind.

3. Kather nach den Ansprüchen 1 und 2, dadurch gekennzeichnet, daß die Schutzkappe (10) den Spitzkegel (8) mit Abstand übergreift und im Bereich des Spitzkegels (8) eine parabelförmige Körperform (geschlossener Kappenkopf (10a) mit Durchflußöffnung (11)) hat.

4. Katheter nach den Ansprüchen 1 bis 3, dadurch gekennzeichnet, daß die Durchflußöffnung (11) in der Schutzkappe (10) in Längsrichtung des Katheterschlauches (2) mit Abstand hinter der Durchflußöffnung (9) des Spitzkegels (8) liegt.

5. Katheter nach den Ansprüchen 1 bis 4, dadurch gekennzeichnet, daß der Spitzkegel (8) an dem Stirnende des Katheterschlauches (2) befestigt, vorzugsweise durch Kleben oder Anvulkanisieren festgelegt ist.

6. Katheter nach den Ansprüchen 1 bis 4, dadurch gekennzeichnet, **daß** der Spitzkegel (8) an den Katheterschlauch (2) einstückig angeformt ist.

7. Katheter nach den Ansprüchen 1 bis 6, dadurch gekennzeichnet, **daß** der Spitzkegel (8) aus einem weicheren Kunststoffmaterial als die Schutzkappe (10) besteht.

8. Katheter nach den Ansprüchen 1 bis 7, dadurch gekennzeichnet, **daß** die Schutzkappe (10) mit ihrem zylindrischen, sich an den gewölbten Kappenkopf (10a) anschließenden Kappenmantel (10b) auf den Katheterschlauch (2) aufgeschoben und darauf lagemäßig festgelegt ist.

9. Katheter nach den Ansprüchen 1 bis 8, dadurch gekennzeichnet, **daß** die Schutzkappe (10) im Bereich ihres Kappenmantels (10b) eine umlaufende nach außen vorstehende Fixierwulst (12) hat.

**Claims**

1. Implantable, arterial catheter, in particular an injection catheter, whith an injection port consisting of a shell with a tightly sealed pierceable membrane and a fixing edge on its open shell side and a catheter tube fixed at one end to the injection port, which has a nonreturn valve on the other end, characterized in that the non-return valve (3) is formed by a pointed cone arranged at the front end of the catheter tube (2) with a flow aperture (9) provided in the point of the cone and by a protective cap (10) having a flow aperture (11), overlapping the pointed cone (8) and the catheter tube end, fixed to the catheter tube (2) towards the front side in the region of the pointed cone.

2. Catheter according to Claim 1, characterized in that the flow aperture (9) in the pointed cone (8) and the flow aperture (11) in the protective cap (10) are formed by slits which open and close according to fluid pressure depending on the fluid introduced via the injection port (1) into the catheter tube (2).

3. Catheter according to claims 1 and 2, characterized in that the protective cap (10) overlaps the pointed cone (8) with a clearance and has a parabolic shape (integral cap head (10a) with flow aperture (11) in the area of the pointed cone (8).

4. Catheter according to Claims 1 to 3, characterized in that the flow aperture (11) in the protective cap (10) lies in the longitudinal direction on the catheter tube (2) at a distance behind the flow aperture (9) in the pointed cone (8).

5. Catheter according to Claims 1 to 4, characterized in that the pointed cone (8) attached to the front end of the catheter tube (2) is fixed preferably by bonding or moulding on.

6. Catheter according to Claims 1 to 4, characterized in that the pointed cone (8) is moulded onto the catheter tube (2) as a single unit.

7. Catheter according to Claims 1 to 6, characterized in that the pointed cone (8) is made from a softer plastic material than the protective cap (10).

8. Catheter according to Claims 1 to 7, characterized in that the protective cap (10) with its cylindrical cap cover joined to the domed cap head (10a) is pushed onto catheter tube (2) and fixed in position.

9. Catheter according to Claims 1 to 8, characterized in that the protective cap (10) has a circumferential fixing bead (12) protruding outwards in the region of the cap cover (10b).

**Revendications**

1. Cathéter artériel implantable, tout particulièrement pour injection, comprenant
une pièce d'injection (port), composée d'une chambre, d'une membrane transperçable, fermant le côté ouvert de la chambre, ainsi que d' un bord de fixation, et un tuyau de cathéter, fixé à une extrémité à la pièce d'injection et pourvu, à l'autre extrémité, d'une soupape de retenue,
caractérisé en ce que la soupape de retenue (3) est formée par un cône pointu (8), disposé à l'extrémité frontale du tuyau de cathéter (2), une ouverture d' écoulement (9) étant prévue dans dans la pointe du cône, et par un capuchon de protection (10), qui emboîte le cône pointu (8) et l'extrémité du tuyau de cathéter (2), sur lequel il est posé fixement, et présente une ouverture d'écoulement (11) dans le secteur du cône pointu, côté frontal.

2. Cathéter selon revendication 1,
caractérisé en ce que l'ouverture d'écoulement (9), prévue dans le cône pointu (8) et l'ouverture d'écoulement (11) équipant le capuchon (10) consistent en fentes s'ouvrant et se fermant sous l'action de la pression exercée par le liquide, en dépendance du liquide introduit par l'intermédiaire de la pièce d'injection (1) dans le tuyau du cathéter.

3. Cathéter selon les revendications 1 et 2, caractérisé en ce que le capuchon de protection (10) emboîte à intervalle le cône pointu (8) et présente un corps parabolique dans le secteur du cône pointu (8) (tête de capuchon fermée (10a) avec ouverture d'écoulement (11)).

4. Cathéter selon les revendications 1 à 3, caractérisé en ce que l'ouverture d'écoulement (11) est située dans le capuchon de protection (10) dans le sens longitudinal du tuyau de cathéter (2), à distance, en arrière de l'ouverture d'écoulement (9) du cône pointu (8).

5. Cathéter selon les revendications 1 à 4, caractérisé en ce que le cône pointu (8) est fixé à l'extrémité frontale du tuyau de cathéter (2), de préférence par collage ou vulcanisation.

6. Cathéter selon les revendications 1 à 4, caractérisé en ce que le cône pointu (8) est formé, d'une pièce, solidairement au tuyau du cathéter (2).

7. Cathéter selon les revendications 1 à 6, caractérisé en ce que le cône pointu (8) est exécuté avec une matière plastique plus molle que celle employée pour le capuchon de protection (10).

8. Cathéter selon les revendications 1 à 7, caractérisé en ce que le manteau cylindrique (10b) du capuchon de protection (10), faisant suite à la tête bombée (10a) du capuchon, est glissé et positionné fixement sur le tuyau de cathéter (2).

9. Cathéter selon les revendications 1 à 8, caractérisé en ce que le capuchon de protection (10) présente, dans le secteur de son manteau (10b), un renflement de fixation circulaire (12) faisant saillie vers l'extérieur.

Fig. 1

Fig. 2

Fig. 3

Fig. 4